# EUROPEAN PATENT SPECIFICATION

(11) **EP 2 801 254 B1**
(45) Date of publication and mention of the grant of the patent: **24.06.2015**
(21) Application number: 14162349.6
(22) Date of filing: 28.03.2014
(51) Int. Cl.: A01K 47/00, A01K 67/033, B65D 85/50

(54) **Device for confining Bumblebees, use of a laminate material for confining bumblebees and method for confining bumblebees**
Vorrichtung zur Eingrenzen von Hummeln, Verwendung eines Mehrschichtmaterials zum Eingrenzen von Hummeln sowie Verfahren zum Eingrenzen von Hummeln
Dispositif pour confiner des bourdons, utilisation d'un matériau stratifié pour confiner des bourdons et procédé de confinement de bourdons

(30) Priority: 07.05.2013 NL 2010768
(43) Date of publication of application: 12.11.2014
(73) Proprietor: Koppert B.V., 2651 BE Berkel en Rodenrijs (NL)
(72) Inventor: Huvermann, Remco Walter, 2651 BE Berkel en Rodenrijs (NL); Muijt, Edwin, 2651 BE Berkel en Rodenrijs (NL); Sima, Peter, SK - 940 01 Nové Zámky (SK); Németh, Richard, SK - 940 01 Nové Zámky (SK)
(74) Representative: Patentwerk B.V.

(56) References cited:
- WO-A2-98/21934
- US-A- 3 408 668
- US-A- 5 190 879
- Koppert Biological: "NATUPOL", , 31 December 2013 (2013-12-31), XP002719117, Retrieved from the Internet: URL:http://www.koppert.nl/producten/bestui ving/producten-bestuiving-hommels/detail/n atupol/ [retrieved on 2014-01-22]

## Description

The present invention relates to the field of bumblebee handling in particular in relation to bumblebee rearing, storage, transport and at the pollination location, see e.g. document WO-A-98/21934.

Bumblebees are beneficial insects widely used as pollinators in agriculture, including horticulture, vegetable and fruit farming and seed propagation. In view of their important role as pollinators they are mass produced by specialized companies. In general a typical bumblebee population used for pollination comprises about 40-250 workers, a single mature queen (the founder queen) and a multitude of eggs, larvae and pupae. Possibly a number of young queens and/or drones (in a premature life stage and/or in a mature life stage) may also be present. Such populations are reared in rearing units by isolating young queens from a population, allow them to mate and hibernate and allowing them to lay eggs in a rearing unit. When workers are present the growing population may be confined in the same or a differing rearing unit formed by a plastic box closed with a plastic lid.

At full development the bumblebee population may be transferred to a nestbox having a volume similar to or different from the rearing unit and is transported to the agricultural sites where the pollinating activity is required. Transport to such locations may take an additional 1-4 days (sometimes up to 7 days) and may be by any suitable transport means including road transport, water transport and/or air transport.

Typically the development time of a population from the moment of installation of the hibernated queen until shipping the hive for pollination purpose is about 9-12 weeks. The development of the colony continues during transport and the pollination period at the agricultural site. Typically the development time of a population from shipping the hive until emergence of adult queens and drones is about 1-3 months.

In view of the fact that bumblebee workers have excellent gnawing capabilities combined with their ability to sting, securely confining bumblebee populations is an important requirement during rearing, storage and transport. Secure confinement is also of importance at the pollination location for example for relocation of active colonies or during periods when the crop is chemically treated.

In view of the above, escape of bumblebees from their confining structure should be minimized and preferably must be prevented at all times in order to secure safety of workers involved with the handling of the bumblebee product and/or to maintain quality of the bumblebee product. This puts stringent requirements on the characteristics of the materials used for structures, such as nest boxes or other packaging structures, in which bumblebees are confined during the process of rearing and/or storage and/or transport and/or at the pollination location.

At present most structures commercially used for confining bumblebees are made of thick-walled plastic to secure bumblebee confinement. It is generally believed within the field that the use of such thick-walled plastic materials is required to securely confine bumblebees. Examples of such bumblebee products using thick-walled plastic materials currently marketed are the Natupol products of Koppert Biological systems (Berkel en Rodenrijs, The Netherlands). The production of bumblebee products utilizing such thick walled plastic materials involves relatively expensive production techniques such as thermoforming or injection molding for forming the plastic parts. Thus it would be beneficial to have additional construction materials available that can be employed more easily, yet which are suitable to confine bumblebees securely.

The inventors of the present invention have now surprisingly found that a laminate comprising a celluloseboard, such as solid paperboard, covered with a 10-500 µm thick layer of a material comprising a number of polymers, said polymeric material having a tensile strength of at least 25 MPa and a microhardness of at least 80MPa, is suitable as a construction material for a device, such as a nestbox, for confining a bumblebee population comprising workers. Such a construction material is easy to employ. For example bumblebee confining structures may be formed by folding a designed blank formed from the laminate into a designed configuration and fixing the blank in said configuration. In addition, tests have shown that structures constructed from such a construction material are sufficiently suitable for securely confining bumblebee populations comprising workers.

The invention thus according to a first aspect relates to a device, such as a nest box, for confining bumblebee *(Bombus* spp.) workers. The device comprises a number of barriers forming a number of chambers suitable for confining bumblebee workers. The barriers comprise a laminate comprising a celluloseboard, preferably solid paperboard, covered with a 10-500 µm thick layer of a material comprising a number of polymers, the polymeric material, said polymeric material having a tensile strength of at least 25 MPa and a microhardness of at least 80 MPa. According to preferred embodiments of the device a layer of polymeric material preferably is an outer surface layer of the laminate, and a side of the laminate where polymeric material is located preferably is faced to the space of the number of chambers where bumblebee workers may be present.

The device according to the invention is suitable for confining bumblebee workers. The number of bumblebee workers that may be confined in the device may start at a single individual and may range up to 350 or even more. Typically populations of bumblebees used for pollination or reared for such purposes may comprise 40-250 workers or even more. In view of this, according to certain embodiments the device is suitable for confining over 40 workers, such as about 40-350, preferably 40-300, more preferably 40-250 workers.

It should be understood that the bumblebee workers may form part of a bumblebee population comprising bumblebee individuals of other classes such as a mature queen (the founder queen), eggs, larvae and/or pupae. Possibly a number of young queens and/or drones (both in a premature life stage and/or in a mature life stage) may also be present.

According to certain embodiments the device is suitable to confine the bumblebee workers during a period of at least 4 days, such as at least 7 days, at least 10 days, at least 14 days or at least 16 days. The time period of confinement of bumblebee workers may extend up to the complete transport period (usually 1-4 days but sometimes extended to 1-7 days), or even the complete rearing period (up to 12 weeks, such as up to 10 weeks).

The device may consist of or comprise a structure, such as a structure comprising a number of chambers, suitable for confining bumblebee workers. As used in the present description the term "structure" refers to anything composed of parts arranged together in some way. By confining the bumblebees they are shut in and/or kept in the structure and thus prevented from leaving the structure. The device preferably is suitable as a nestbox for a bumblebee population comprising workers. The skilled person will readily understand the geometric requirements for devices and/or structures suitable for confining bumblebee workers, such as nestboxes. The term "nestbox" is to be construed as including rearing units, transport units and hives. Within the context of the present description and the appended claims "a number of" should be construed as meaning one or more, whenever this term is used, unless otherwise stated, such as 1, 2, 3, 4, 5, 6, 7 etcetera.

According to particular embodiments, the confining chambers comprise a number of sections having an angular geometry. Chambers comprising sections having an angular geometry may comprise a number of corners formed by adjoining barriers having a flat surface or may comprise a number of barriers having a flat surface that changes direction under an angle, in particular a right angle or an oblique angle. Chambers comprising sections having an angular geometry may be formed in structures having an angular geometry, such as box-like structures. It is known in the art that bumblebee workers gnaw in particular in corners of chambers wherein they are confined. Thus in particular for bumblebee confining devices comprising chambers having sections of angular geometry, the provision of additional construction materials is beneficial.

A flat surface is to be construed as including an "essentially flat" surface. An "essentially flat surface" being a surface that essentially is flat with only non-essential or minor deviations from a perfectly flat surface. Corners include locations where converging flat surfaces of different barriers meet (under an angle) at an intersection line or where the direction of a flat surface of a barrier changes directions (under an angle). The angle between converging barriers or of the directional change of the flat surface of a barrier in particular may be a right angle (90°), an oblique angle (not 90° or multitude of 90°), such as an acute angle (> 0°, < 90°) or an obtuse angle (>90°, <180°), or is a reflex angle (>180°, <360°). Typically the angle may be selected from an angle between 10° and 170°, such as 20-160°, 30-150°, 40-130°, 50-120°, 60-120°, 70-120°, 80-110°, or between 190° and 350°, such as 200-340°, 210-330°, 220-320°, 230-310°, 240-300°,250-290°,260-280°.

The chambers should be suitable for confining the bumblebees and thus should not have any permanent openings which would allow passage of bumblebee individuals, in particular workers but preferably also queens and drones, when present. For ventilation the structure may comprise a number of ventilation openings. Ventilation openings may for example be formed by covering an opening with bars and/or a mesh to prevent escape of bumblebee individuals. In addition, the structure may comprise a number of closable passages such as exits and/or entries to allow entry and/or exit of bumblebee individuals when this is required, for example to allow their pollinating activity.

In the structure the number of chambers is formed from a number of barriers. In general a number of, usually a plurality of, barriers will define a chamber by surrounding a void. Any barrier suitable to surround a number of voids, thus forming a number of chambers, and suitable to confine bumblebee individuals, in particular bumblebee workers, may be used. In general walls or other wall-like barriers are preferred. As the skilled person will understand, walls and wall-like barriers are barriers having a length and width greater than the thickness. According to embodiments of the invention the length and width are at least half an order of magnitude, and preferably an order of magnitude, greater than the thickness. Numbers may be considered to differ half an order of magnitude, if the result of the division of the larger number divided by the smaller number is ≥ 5. Numbers may be considered to differ an order of magnitude, if the result of the division of the larger number divided by the smaller number is ≥ 10. Within the context of the present description and the appended claims "a plurality" should be construed as meaning two or more, whenever this term is used, unless otherwise stated, such as 2, 3, 4, 5, 6, 7 etcetera.

According to the invention the barriers comprise, and according to certain embodiments consist of, a laminate comprising a celluloseboard, covered with a 10-500 µm thick layer of a material comprising a number of polymers, said polymeric material having a tensile strength of at least 25 MPa and a microhardness of at least 80 MPa. Such a construction material is easy to employ yet such a construction material is sufficiently suitable for securely confining bumblebee populations comprising workers.

The term "cellulose board" should be construed as referring to any board shaped material consisting essentially of cellulose fibres with optionally a number of added constituents to affect the quality of the board and its fitness for intended end use. For example a number of water-resistant additives may be added. The cellulose board preferably is selected as solid paperboard (solid fibre board) or fluted or corrugated paper board (fluted or corrugated fibre board). The use of solid paperboard is most preferred. According to standards set by the paper industry "paperboard" generally refers to paper material having a product sheet weight (grammage) of over 150 g/m². According to the invention the cellulose board may have a mass of at least 200 g/m², preferably 200-3000 g/m², such as 200-2000 g/m², more preferably 550-1200 g/m².

The cellulose board is covered by a 10-500 µm thick layer of a material comprising a number of polymers, the polymeric material, thus forming a laminate. A "laminate" should be construed as a material comprising a plurality of sheets and/or layers bound together. The layer of the polymeric material may be bound directly to the cellulose board or alternatively a number of intermediate layers may be present. For example the layer may be applied to a paper sheet (grammage <150 g/m²) and the paper sheet may be bound to the cellulose board. Additional intermediate layers may be present for example between a paper sheet and the layer of polymeric material. Bonding of layers may be by any suitable means known in the art, such as by using suitable adhesives. Alternatively the polymeric material may be applied to the cellulose board or a layer covering the cellulose board when in a state allowing adhesion, such as in a melted state or a partially melted state. According to a further alternative, curable compounds may be applied to the cellulose board or a layer covering the cellulose board and allowed to cure. For example resins or lacquers may be cured on the surface of the cellulose board or on a layer covering the cellulose board.

According to certain embodiments, a layer of polymeric material preferably is an outer surface layer of the laminate. However, it is not required that the layer of polymeric material is an outer surface. Additional layers may cover the layer of polymeric material.

The term "covered" and related terms such as "covering", "covers" etcetera indicate that the layer of polymeric material overlays the cellulose board sheet. The covering of the polymeric material will be in the form of a layer of the polymeric material. Such a layer of the polymeric material may be a continuous layer covering the whole surface of a side of the cellulose board. In alternative embodiments only part of the surface of a side of the cellulose board may be covered with a layer of a polymeric material. The covering may be on a single side or on both sides of the cellulose board.

Whether or not in the laminate the whole surface of a side of the cellulose board or only part of the surface is covered with a layer of polymeric material, it is preferred that the layer of polymeric material covers the cellulose board in any corner of the chambers. The covering layer of polymeric material in the corners preferably is present up to 20 cm, up to 15 cm, up to 10 cm, up to 5 cm, up to 2 cm or up to 1 cm from the intersection line of converging barriers or from the line where a barrier's flat surface changes direction.

According to preferred embodiments the thickness of the polymeric material in the laminate is selected from 8-15, 10-15, 10-20, 10-30, 10-40, 10-50, 10-60, 10-70, 10-80, 10-90, 10-100, 10-110, 10-120, 10-130, 10-140, 10-150, 10-160, 10-170, 10-180, 10-190, 10-200, 10-250, 10-300, 10-350, 10-400, 10-450 µm. The preferred range of the thickness is 12-100 µm, more preferably 12-80 µm. It should be understood that any upper limit presented may be used as a lower limit for alternative intervals. For example a range of 15-80 µm is also within the scope of the invention. Selections of the thickness of the polymeric material within the intervals mentioned results in a laminate with suitable properties. The use of foils of the polymeric material having the indicated thickness is preferred according to certain embodiments of the invention.

The polymeric material covering the cellulose board has a tensile strength of at least 25 MPa. Tensile strength is a material property well known in the art and refers to the maximum stress that a material can withstand while being stretched or pulled before failing or breaking. Alternative terms used are Ultimate tensile strength or ultimate strength. According to a preferred embodiment the tensile strength is measured according to ASTM D882 (standard Test Method for Tensile Properties of Thin Plastic Sheeting), more preferably ASTM D882-12. Tensile strength values for commercially relevant polymers may also be derived from Callister (2003), Osborne et al (1992), the Modern Plastics Encyclopedia '96, (McGraw-Hill Companies) or from other sources readily available to the skilled person.

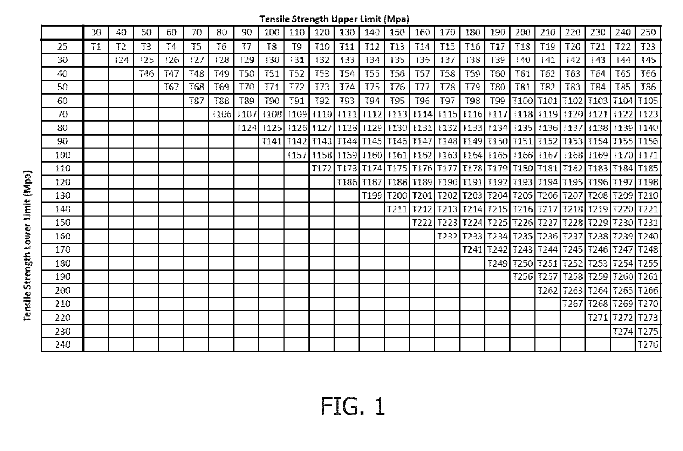

According to certain embodiments, the tensile strength of the polymeric material has a value greater than a value selected from 30, 40, 50, 60, 70, 80, 90, 100, 110, 120, 130, 140, 150, 160, 170, 180, 190, 200, 210, 220, 230 MPa. There is no particular upper limit for the value of the tensile strength other than what is practically achievable. However, it has been found that materials having a value for the tensile strength below 250 MPa, such as below 240 MPa are sufficiently suitable within the context of the invention. According to certain embodiments such values for the tensile strength of below 250 MPa, such as below 240 MPa are preferred. Thus according to certain embodiments of the invention the tensile strength of the polymerised material may be within any of the ranges T1-T276 defined by combinations of lower and upper limit values as presented in figure 1.

The polymeric material covering the cellulose board in addition has an indentation micro hardness of at least 80 MPa. Indentation hardness is another material property well known in the art and refers to the resistance of the material to local surface deformation against indentation. It may be measured in tests employing a micro range (9.807•10⁻³ to ≤ 9.807 N) or macro range (>9.807 to ≤ 1176.80 N) test force. The indentation hardness determined for the polymeric material is measured using a micro range test force and thus may be referred to as indentation microhardness or microhardness. According to a preferred embodiment microhardness is measured according to ASTM-E384 (Standard Test Method for Knoop and Vickers Hardness of Materials), more preferably ASTM-E384-11^{ε¹}, using a Vickers indenter. Microhardness values for commercially relevant polymers may also be derived from Baltá Calleja & Fakirov (2000) or from other sources readily available to the skilled person.

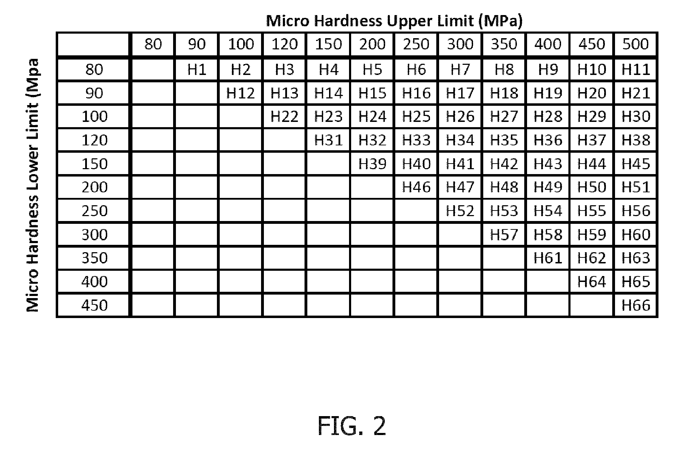

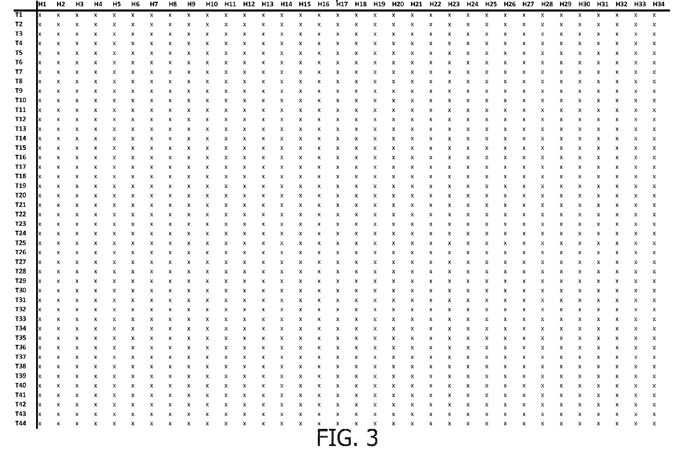

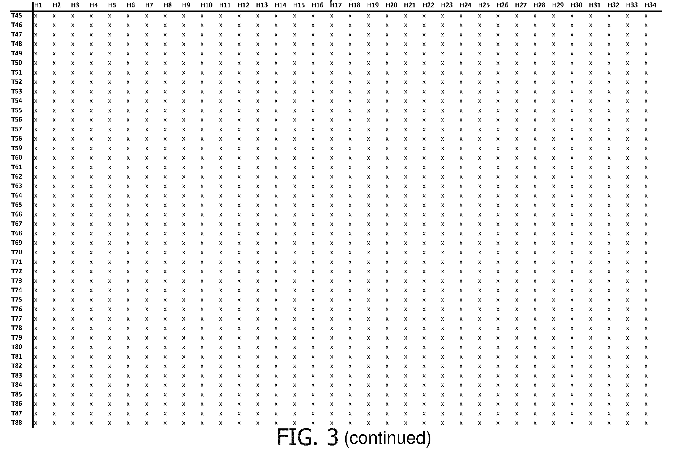

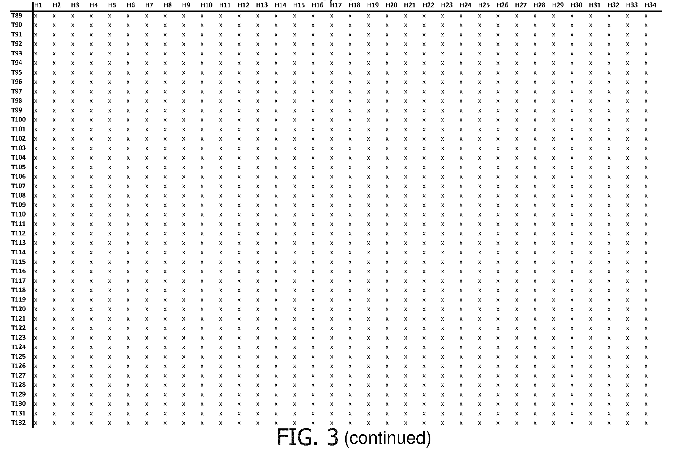

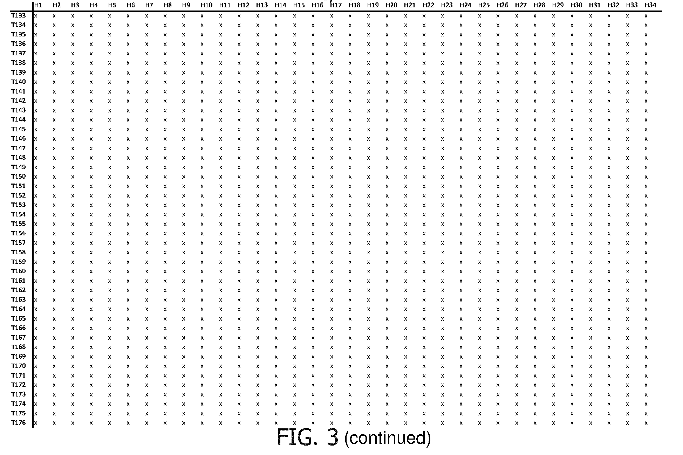

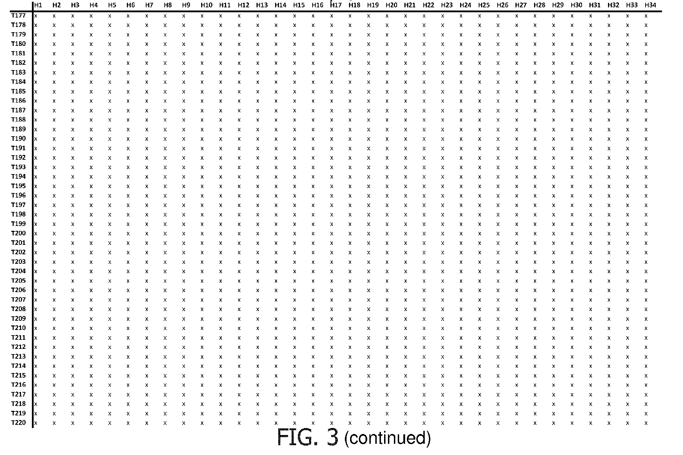

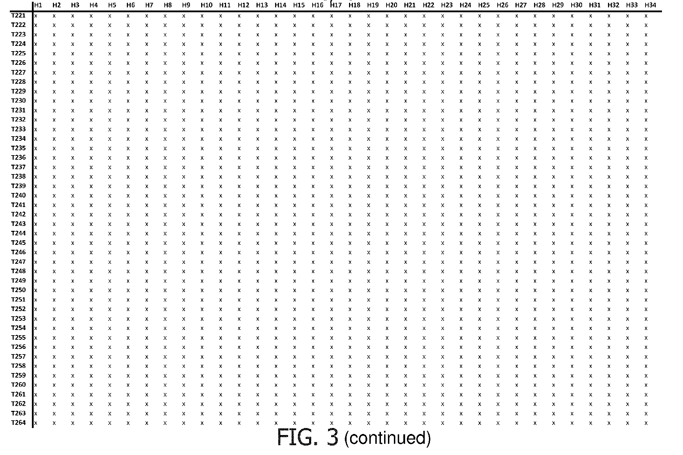

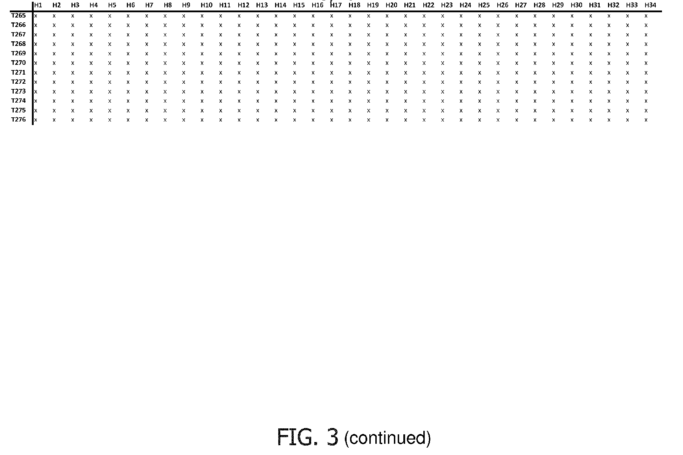

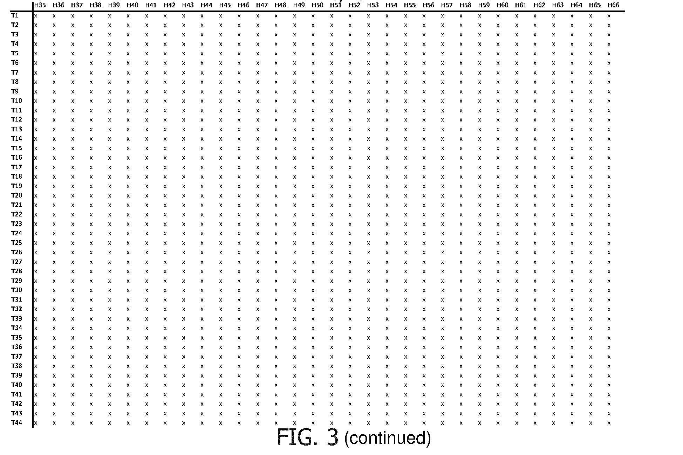

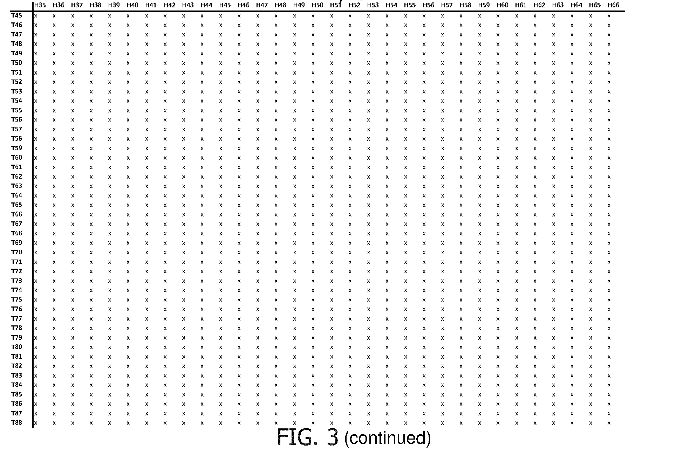

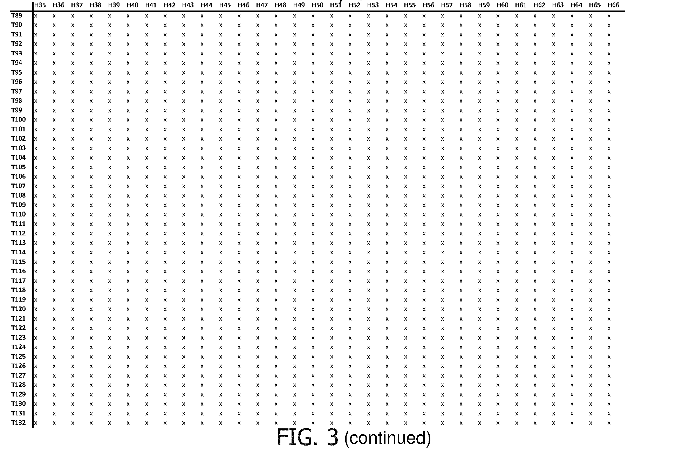

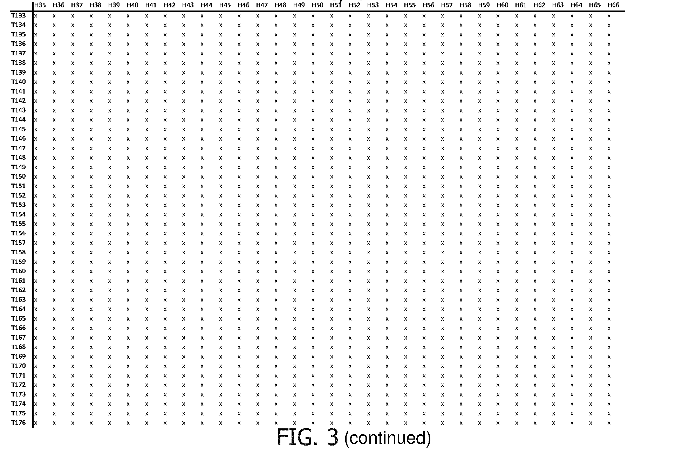

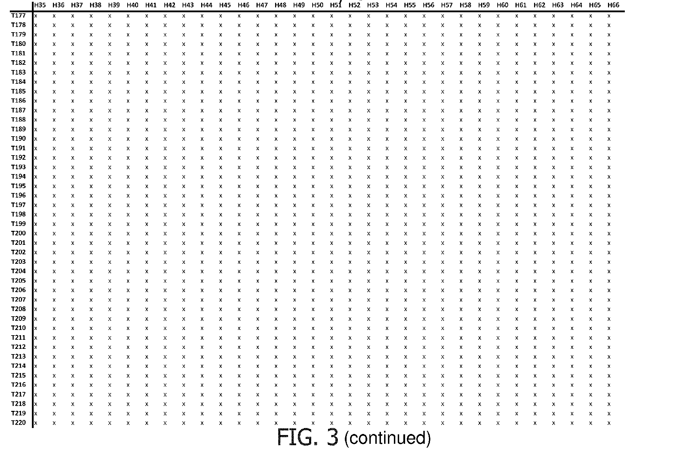

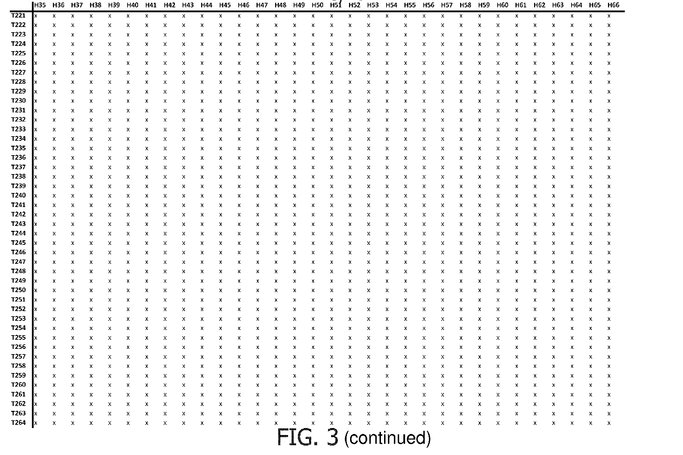

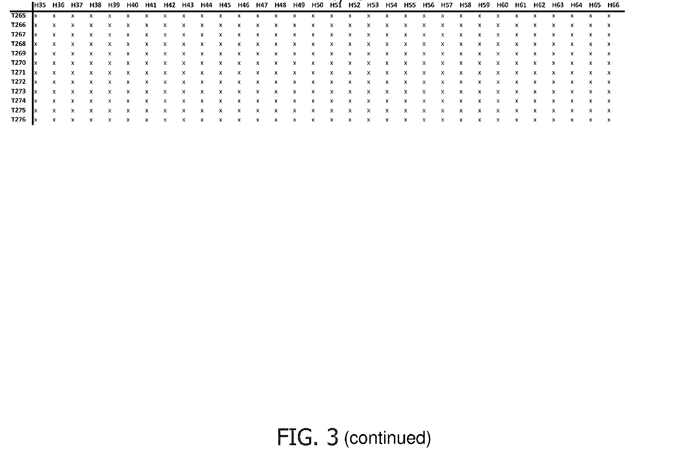

According to certain embodiments, the microhardness has a value greater than a value selected from 80, 90, 100, 120, 150, 160, 170, 180 MPa. There is no particular upper limit for the value of the microhardness other than what is practically achievable. However, it has been found that materials having a value for the microhardness below 300 MPa, such as below 290 MPa, below 280 MPa, below 270, MPa or below 260 MPa are sufficiently suitable within the context of the invention. According to certain embodiments such values for the microhardness of below 300 MPa, such as below 290 MPa, or below 280 MPa are preferred. Thus according to certain embodiment of the invention the microhardness of the polymerised material may be within any of the ranges H1-H66 defined by combinations of lower and upper limit values as presented in figure 2. In the present invention polymeric materials may be selected having a microhardness value within any of the ranges H1-H66, as presented in figure 2, combined with a tensile strength value within any of the ranges T1-T276, as presented in figure 1, in particular in the combinations as presented in figure 3.

In the device according to the invention a layer of polymeric material preferably is an outer surface layer of the laminate and the polymeric material layer side of the laminate preferably is faced to the space of the number of chambers where bumblebee workers may be present. In this configuration the layer of polymeric material is the laminate layer that the bumblebee workers encounter first, when they try to gnaw trough the construction material of the barriers. In this configuration damage to the cellulose board is prevented or at least reduced which improves the durability of the nestbox.

According to certain embodiments wherein a layer of polymeric material is an outer surface layer of the laminate and the polymeric material layer side of the laminate is faced to the space of the number of chambers where bumblebee workers may be present, the microhardness of the cellulose board is lower than the microhardness of the polymeric material. This creates a construction wherein a relatively hard material covers a relatively softer material. This further improves resistance to gnawing in particular when relatively thin layers (12-100 µm, such as 12-80 µm) of polymeric material are used. According to such embodiments the microhardness of the cellulose board may have a value of 90-10%, such as 80-20%, 70-30%, 60-40% or 50-40% of the microhardness value of the polymeric material.

Regarding the bumblebees for which the device is suitable for confinement, it may be noted that these may be selected from any *Bombus spp* such as in particular *B. terrestris,* more in particular *B. terrestris terrestris, B. terrestris audax, B. terrestris dalmatinus* or *B. terrestris sassaricus, B. canariensis* (or alternatively *B. terrestris canariensis), B. impatiens, B. vosnesenskii, B. ignitus, B. diversus, B. occidentalis,* including any sub-species.

The polymeric material covering the cellulose board comprises a number of polymers. The polymer may be selected from any natural or synthetic polymer. As stated previously, within the context of the present description and the appended claims "a number of" should be construed as meaning one or more, whenever this term is used, unless otherwise stated, such as 1, 2, 3, 4, 5, 6, 7 etcetera. Thus the polymeric material may comprise a single polymer or a plurality of polymers forming a blend. The chemical structure of the polymer is not essential, as long as the polymeric material obtains the required mechanical properties of tensile strength and microhardness. The polymeric material may comprise essentially linear polymers and/or may comprise cross-linked polymer chains. Apart from polymers, additives such as lubricators, heat stabilizers, light stabilizers, plasticizers, fillers, flame retardants, fragrances, impact modifiers, reinforcements, pigments etcetera may be present in the polymeric material. It is preferred that polymers constitute the major part of the polymeric material.

Polymers combining suitable values for tensile strength and microhardness, and which may thus be chosen within the selection of the number of polymers for the polymeric material, may be selected from polyamides (PA), such as PA5/10, PA6, PA 6/4, PA6/6, PA6/9, PA 6/10, PA6/12, PA6/66, PA10/10, PA11, PA12, PA12/12, PA4/6, PA66/610, PA6I, PA mXD6, bi-axially oriented polyamide (boPA) and oriented polyamide (oPA); polystyrene (PS), such as high/impact polystyrene (HIPS) and oriented polystyrene (OPS); polypropylene (PP), such as cast polypropylene (CPP), oriented polypropylene (OPP) and bi-axially oriented polypropylene (BOPP); polyvinylchloride (PVC), such as Polyvinylidene chloride (PVDC), unplasticized polyvinyl chloride (uPVC) and plasticized polyvinyl chloride (pPVC); polyethylene terephthalate (PET), such as amorphous polyethylene terephthalate (APET), poly terephthalate glycol (PETG), metallized polyethylene terephthalate (MPET), oriented polyethylene terephthalate (oPET) and bi-axially oriented polyethylene terephthalate (boPET); polycarbonate (PC); polylactic acid (PLA); polyhydroxyalkanoate (PHA), such as poly-3-hydroxybutyrate (P3HB), poly-4-hydroxybutyrate (P4HB), polyhydroxyvalerate (PHV), polyhydroxyhexanoate (PHH), polyhydroxyoctanoate (PHO), poly-3 hydroxy butyrate-co-3 hydroxyhexanoate (PHBH) and poly-3-hydroxy butyrate-covalerate copolymer (PHBV); thermoplastic starch (TPS); cellulose based plastics, such as cellulose-hydrate, cellulose acetate (CA), cellulose acetate butyrate (CAB), cellulose propionate (CP), cellulose butyrate (CB), cellulose acetate propionate (CAP), and cellulose triacetate (CTA); polyethylene furanoate (PEF), polybutylene succinate (PBS); polybutylene succinate adipate (PBSA); polybutylene terephthalate (PBT); polymethyl methacrylate (PMMA), acrylonitrile butadiene styrene (ABS); polyether ether ketone (PEEK); polyoxymethylene (POM); polyphenylene ether (PPE); polyphenylene oxide (PPO); polyphenylene sulfide (PPS); Polymethylepentene (PMP); styrene acrylonitrile (SAN); acrylonitrile styrene acrylate (ASA); polyurethane (PU); alkyds; acrylics; epoxies (EP); allylics, such as diallyl phthalate (DAP), diallyl iso-phthalate (DAIP) and allyl diglycol carbonate (ADC); melamine formaldehyde (MF); urea formaldehyde (UF); phenol formaldehyde (PF); vinylester.

Preferably one or more of the number of polymers for the polymeric material may be selected from a polyamide (PA), a polystyrene (PS), a polyethylene terephthalate (PET), a polypropylene (PP), a polyvinylchloride (PVC), a polylactic acid (PLA) or a polyhydroxyalkanoate (PHA), such as poly-3-hydroxy butyrate-covalerate copolymer (PHBV).

The polymeric material may comprise an individual polymer from the above lists or a blend of polymers. The use of PET (in particular in the 10-50 µm thickness range, preferably in the 10-20 µm thickness range) or OPP (in particular in the 10-80 µm thickness range) is preferred. It has been shown that the use of laminates comprising covering layers of these polymers have good resistance against gnawing of bumblebee workers while these polymers also are readily available and may be processed easily.

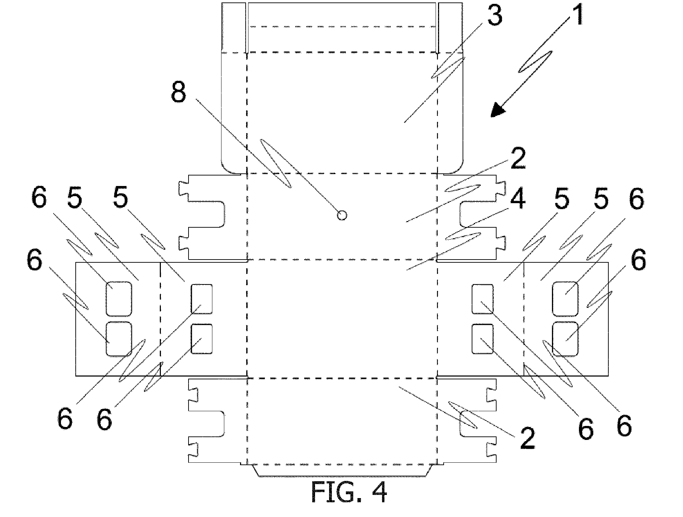

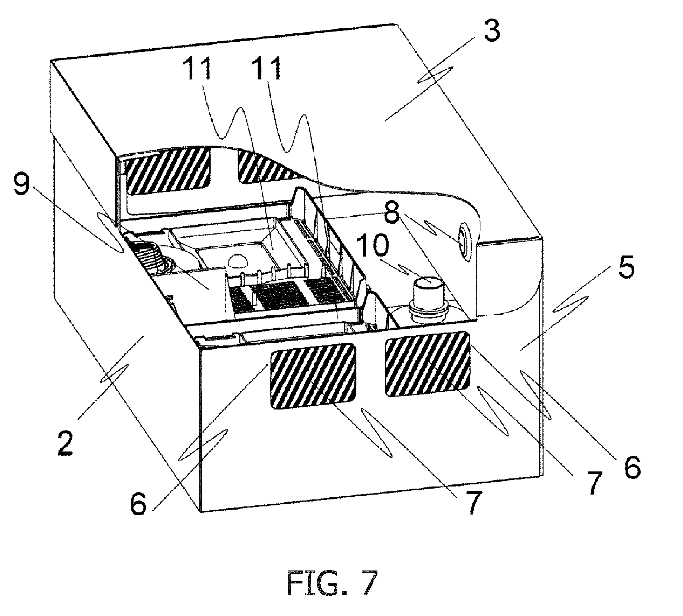

According to a preferred embodiment, at least part of the device is folded from a blank formed from the laminate. The use of blanks for constructing structures is known in the art. In general a designed layout of the blank is cut from a sheet of material. Cutting may be achieved by any suitable means. After cutting the blank is folded in the designed conformation and fixed to that conformation. Fixing may be achieved by any suitable means including staples, glue (in particular hotmelt), clips, adhesive tape, straps, or the design of the blank may comprise locking parts. According to an embodiment of the invention a blank as presented in figure 4 may be used. From this blank a nestbox as presented in figure 7 may be formed by folding it in the designed configuration and fixing it in the designed configuration.

A further aspect of the invention relates to the use of a laminate comprising a celluloseboard, preferably solid paperboard, covered with a 10-500 µm thick layer, preferably as an outer surface covering layer, of a material comprising a number of polymers, said polymeric material having a tensile strength of at least 25 MPa and a indentation microhardness of at least 80MPa, as a construction material for a device, such as a nestbox, for confining bumblebee workers, wherein in said use a layer of polymeric material preferably is faced to spaces in the device where bumblebee workers may be present.

The details of the specific features of the laminate and of preferred embodiments will be clear from the above description in relation to the device of the invention which uses the same laminate.

Yet a further aspect of the invention relates to a method for confining bumblebee workers. Said method comprises the steps of:
- providing a laminate comprising celluloseboard, preferably solid paperboard, covered with a 10-500 µm thick layer, of a material comprising a number of polymers, said polymeric material having a tensile strength of at least 25 MPa and an indentation microhardness of at least 80 MPa, wherein a layer of polymeric material preferably is an outer surface layer of the laminate;
- constructing from the laminate a device, such as a nest box, comprising a number of confining chambers suitable for confining bumblebee workers, wherein the device preferably is constructed such that the polymeric layer side of the laminate is faced to spaces where bumblebee workers may be present;
- placing a number of bumblebee workers in confining chambers of the device.

Also for this aspect of the invention the laminate and the device constructed therefrom are key elements. The details of the specific features of the laminate and the device and of preferred embodiments of the laminate and the device will be clear from the above description in relation to the device of the invention which uses the same laminate.

The invention will now be illustrated with reference to the following experiments which make use of certain embodiments of the invention. It should be understood that the invention is not limited to the use of these specific embodiments.

### Experiments

### General outline

*Bombus terrestris* colonies consisting of a minimum of 50 larvae and a minimum of 150 workers (typical: more than 300 workers) are placed in hives folded from a blank as presented in figure 4. The blank (1) is cut (cutting lines are indicated in continuous lines) from a laminate of solid paperboard having a grammage of 850-900 g/m² covered with a polymer foil. In the folded configuration (folding lines are indicated in broken lines) of the blank, the longest walls (2) and the upper (3) and lower plane (4) of the hives are formed by a single layer of laminate and the shortest walls (5) of the hives are made of two layers of the laminate. The shortest walls (5) of the hives each have two ventilation openings (6), which in the fully assembled hive are covered with a mesh (7) having square openings of 2mm x 2mm. One of the longest walls comprises an entry and exit hole (8) which is closable with a plug.

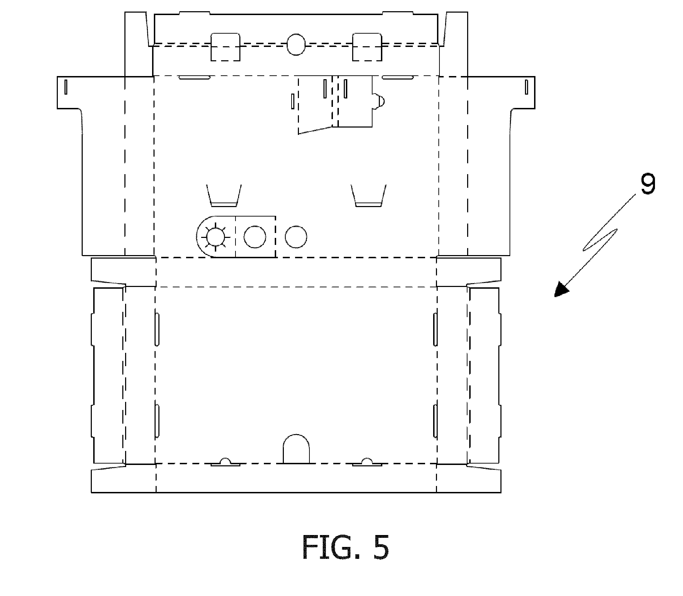

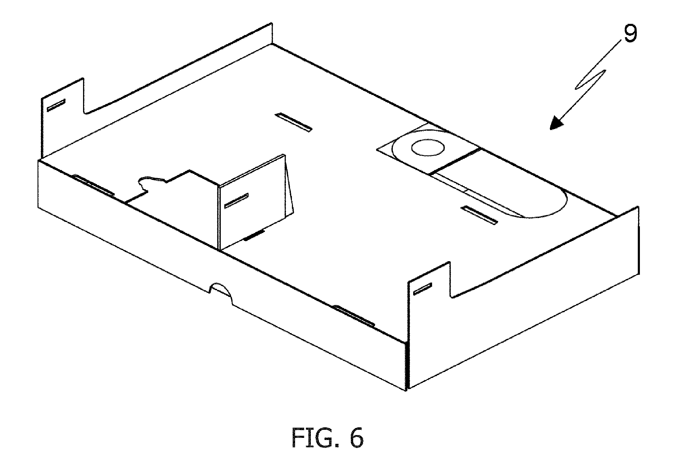

To facilitate installation of a bag comprising sugar water (approx. 2 kilogram of sugar solution) serving as nutrition for the bumblebees the blank of figure 5 is designed and cut from corrugated paperboard (cutting lines are indicated in continuous lines, folding lines are indicated in broken lines having short intervals, cut folds are indicated in broken lines having long intervals). This blank may be folded in the interior element (9) presented in figure 6. The main function of the interior element (9) is to securely fix the rearing bottoms and the sugar bag.

The fully assembled hive is presented in figure 7. After folding, the blanks are fixed in the designed configuration with staples. In view of bumblebee escapes in earlier preliminary experiments, as a safety precaution, aluminium tape was applied on the corners for minimum 20 mm on the outside of hives used in the tests. The sugar solution is accessible via a feeding tip (10) where one end of a fibrous fuse hangs in the sugar solution. The sugar solution in principle provides sufficient carbohydrates for the colony to sustain itself during the time of the experiment. The workers and larvae are transferred into the hives on a rearing bottom (11). The presence of larvae is a stimulus for workers to search for pollen and nectar in order to cover the colony's nutrient requirements.

The hives containing the bumblebee colonies are loaded on a pallet and placed outdoor, sheltered from sight, rain, direct sunlight and wind, at temperatures between 7°C and 28°C. Pollen is provided in limited quantities, sufficient for only 5 days.

At least every 72 hours the hives are checked for visible damage on the outside. Critical damage in this test is defined as an opening with an inscribed circle of minimum 6 mm diameter. When critical damage is found, the test is stopped and the time between placing the bumblebees in the hive and the first observation of the critical damage is recorded. After freezing the hives, the interior of the hive is also inspected.

### Experiment 1A: PET coated paperboard

The laminate consists of the paperboard with a layer of 12 µm PET coated on each side. PET is a polymer having a reported tensile strength of around 172 MPa and a micro hardness of around 200 MPa. The test batch contained 2 hives, of which 0 showed critical damage 14 days after placing the bumblebees in the enclosure. The visual inspection of the interior of the hives revealed no damage that could potentially progress to critical damage.

### Experiment 1B: OPP coated paperboard

The laminate consists of the said paperboard with a layer of 80 µm OPP coated on each side. OPP is a polymer having a reported tensile strength of around 172-207 MPa and a micro hardness of around 80 MPa. The test batch contained 2 hives, of which 0 showed critical damage 14 days after placing the bumblebees in the enclosure. The visual inspection of the interior of the hives revealed no damage that could potentially progress to critical damage.

### Experiment 1C (comparative): PE coated paperboard

The laminate consists of the paperboard with a layer of 12 µm PE coated on each side. PE is a polymer having a reported tensile strength of about 8.3 to 17.2 MPa and a micro hardness of around 25 MPa.

The test batch contained 2 hives, of which 2 showed critical damage in a top corner two days after placing the bumblebees in the enclosure. The visual inspection of the interior of the hives showed that besides the critical damage, adjacent to the aluminum tape the laminate of the corners was removed over the length of the aluminum tape.

The tests show the secure bumblebee confinement in exemplary embodiments of the invention over a period of up to 14 days. In view of the absence of damage on the interior side of the hive that could potentially progress to critical damage, there is a justified confidence that by using the present invention secure bumblebee confinement can be maintained over longer periods of time. This was confirmed by further tests in which 70 of the PET coated hives containing bumblebee colonies were put on transport during a period of 5 days without any bumblebee escapes or signs of exterior damage.

### List of references

Callister, William D. Jr., Materials Science and Engineering an Introduction 6th edition, (2003) John Wiley & Sons,
McGraw-Hill Companies, Modern Plastics Encyclopedia '96, (1995)
Baltá Calleja, F.J., and Fakirov, S., Microhardness of Polymers, (2000) Cambridge University Press
Osborn, Kenton R., and Jenkins, Wilmer A., Plastic Films Technology and Packaging Applications, (1992) Technonomic Publishing Company

## Claims

1. Device, for confining bumblebee (*Bombus* spp.) workers, such as a nest box, comprising a number of barriers (2, 3, 4, 5) forming a number of chambers suitable for confining bumblebee workers, wherein said barriers (2, 3, 4, 5) comprise a laminate comprising a celluloseboard, preferably solid paperboard, covered with a 10-500 µm thick layer of a material comprising a number of polymers, said polymeric material having a tensile strength of at least 25 MPa and an indentation microhardness of at least 80 MPa, wherein a layer of polymeric material preferably is an outer surface layer of the laminate, and wherein a side of the laminate where polymeric material is located preferably is faced to the space of the number of chambers where bumblebee workers may be present.

2. Device according to claim 1, wherein the thickness of the layer of polymeric material is selected from 10-20, 10-30, 10-40, 10-50, 10-60, 10-70, 10-80, 10-90, 10-100, 10-110, 10-120, 10-130, 10-140, 10-150, 10-160, 10-170, 10-180, 10-190, 10-200, 10-250, 10-300, 10-350, 10-400, 10-450 µm.

3. Device according to any of the preceding claims, wherein the cellulose board has a mass of at least 200 g/m², preferably 200-3000 g/m², such as 200-2000 g/m², more preferably 550-1200 g/m².

4. Device according to any of the preceding claims, wherein the tensile strength has a value greater than a value selected from 30, 40, 50, 60, 70, 80, 90, 100, 110, 120, 130, 140, 150, 160, 170, 180, 190, 200, 210, 220, 230 MPa.

5. Device according to any of the preceding claims, wherein the indentation microhardness has a value greater than a value selected from 80, 90, 100, 120, 150, 200, 250, 300, 350, 400, 450, 500 MPa.

6. Device according to any of the preceding claims, wherein the polymeric material comprises one or more polymers selected from polyamide (PA), polystyrene (PS), polyethylene terephthalate (PET), polypropylene (PP), polyvinylchloride (PVC), polylactic acid (PLA) or polyhydroxyalkanoate (PHA), in particular poly-3-hydroxy butyrate-covalerate copolymer (PHBV).

7. Device according to any of the preceding claims, wherein the nest box is folded from a blank (1) formed from the laminate.

8. Device according to any of the claims 1-7, wherein the cellulose board is selected as solid paperboard having a grammage of 825-925 g/m², preferably 850-900 g/m², the layer of polymerized material has a thickness of 10-80 µm, and the polymeric material is selected as polyethylene terephthalate (PET), preferably bi-oriented polyethylene terephthalate (BOPET), or polypropylene (PP), preferably oriented polypropylene (OPP).

9. Use of a laminate comprising a celluloseboard, preferably solid paperboard, covered with a 10-500 µm thick layer, preferably as an outer surface covering layer, of a material comprising a number of polymers, said polymeric material having a tensile strength of at least 25 MPa and an indentation microhardness of at least 80MPa, as a construction material for a device, such as a nestbox, for confining bumblebee workers, wherein in said use a polymeric material side of the laminate preferably is faced to spaces in the device where bumblebee workers may be present.

10. Use according to claim 9, wherein the thickness of the layer of polymeric material is selected from 10-20, 10-30, 10-40, 10-50, 10-60, 10-70, 10-80, 10-90, 10-100, 10-110, 10-120, 10-130, 10-140, 10-150, 10-160, 10-170, 10-180, 10-190, 10-200, 10-250, 10-300, 10-350, 10-400, 10-450 µm.

11. Use according to any of the preceding claims 9-10, wherein the cellulose board has a mass of at least 200 g/m², preferably 200-3000 g/m², such as 200-2000 g/m², more preferably 550-1200 g/m².

12. Use according to any of the preceding claims 9-11, wherein the tensile strength has a value greater than a value selected from 30, 40, 50, 60, 70, 80, 90, 100, 110, 120, 130, 140, 150, 160, 170, 180, 190, 200, 210, 220, 230 MPa.

13. Use according to any of the preceding claims 9-12, wherein the indentation microhardness has a value greater than a value selected from 80, 90, 100, 120, 150, 200, 250, 300, 350, 400, 450, 500 MPa.

14. Use according to any of the preceding claims 9-13, wherein the polymeric material comprises one or more polymers selected from polyamide (PA), polystyrene (PS), polyethylene terephthalate (PET), polypropylene (PP), polyvinylchloride (PVC), polylactic acid (PLA) or polyhydroxyalkanoate (PHA), in particular poly-3-hydroxy butyrate-covalerate copolymer (PHBV).

15. Use according to any of the preceding claims 9-14, wherein a blank (1) is formed from the laminate and the device is folded from the blank and preferably the folded blank (1) is fixed in a folded configuration.

16. Use according to any of the claims 9-15, wherein the cellulose board is selected as solid paperboard having a grammage of 825-925 g/m², preferably 850-900 g/m², the layer of polymeric material has a thickness of 10-80 µm, and the polymeric material is selected as polyethylene terephthalate (PET), preferably bi-oriented polyethylene terephthalate (BOPET) or polypropylene (PP), preferably oriented polypropylene (OPP).

17. Method for confining bumblebee workers comprising:
- providing a laminate comprising celluloseboard, preferably solid paperboard, covered with a 10-500 µm thick layer, of a material comprising a number of polymers, said polymeric material having a tensile strength of at least 25 MPa and an indentation microhardness of at least 80 MPa, wherein a layer of polymeric material preferably is an outer surface layer of the laminate;
- constructing from the laminate a device, such as a nest box, comprising a number of confining chambers suitable for confining bumblebee workers, wherein the device preferably is constructed such that a polymeric layer side of the laminate is faced to spaces where bumblebee workers may be present;
- placing a number of bumblebee workers in confining chambers of the device.

18. Method according to claim 17, wherein the device is a device according to any of the claims 1-7.

## Patentansprüche

1. Vorrichtung zum Eingrenzen von Arbeiterinnen der Hummeln *(Bombus* spp.), wie ein Nistkasten, umfassend eine Anzahl von Barrieren (2, 3, 4, 5), die eine Anzahl von Kammern bilden, die zum Eingrenzen von Arbeiterinnen der Hummeln geeignet sind, wobei die Barrieren (2, 3, 4, 5) ein Laminat umfassen, das eine Celluloseplatte, vorzugsweise stabile Pappe, bedeckt mit einer 10-500 µm dicken Schicht eines Materials umfasst, das eine Anzahl von Polymeren umfasst, wobei das Polymermaterial eine Zugfestigkeit von mindestens 25 MPa und eine Eindrück-Mikrohärte von mindestens 80 MPa aufweist, wobei eine Schicht aus Polymermaterial vorzugsweise eine Außenseitenschicht des Laminats ist, und wobei eine Seite des Laminats, an der sich Polymermaterial befindet, vorzugsweise dem Raum der Anzahl der Kammern gegenüberliegt, worin Arbeiterinnen der Hummeln vorhanden sein können.

2. Vorrichtung nach Anspruch 1, wobei die Dicke der Schicht aus Polymermaterial ausgewählt ist aus 10-20, 10-30, 10-40, 10-50, 10-60, 10-70, 10-80, 10-90, 10-100, 10-110, 10-120, 10-130, 10-140, 10-150, 10-160, 10-170, 10-180, 10-190, 10-200, 10-250, 10-300, 10-350, 10-400, 10-450 µm.

3. Vorrichtung nach einem der vorhergehenden Ansprüche, wobei die Celluloseplatte eine Masse von mindestens 200 g/m², vorzugsweise 200-3000 g/m², wie 200-2000 g/m², insbesondere 550-1200 g/m² aufweist.

4. Vorrichtung nach einem der vorhergehenden Ansprüche, wobei die Zugfestigkeit einen Wert aufweist, der einen Wert ausgewählt aus 30, 40, 50, 60, 70, 80, 90, 100, 110, 120, 130, 140, 150, 160, 170, 180, 190, 200, 210, 220, 230 MPa übersteigt.

5. Vorrichtung nach einem der vorhergehenden Ansprüche, wobei die Eindrück-Mikrohärte einen Wert aufweist, der einen Wert ausgewählt aus 80, 90, 100, 120, 150, 200, 250, 300, 350, 400, 450, 500 MPa übersteigt.

6. Vorrichtung nach einem der vorhergehenden Ansprüche, wobei das Polymermaterial ein oder mehrere Polymere ausgewählt aus Polyamid (PA), Polystyrol (PS), Polyethylenterephthalat (PET), Polypropylen (PP), Polyvinylchlorid (PVC), Polymilchsäure (PLA) oder Polyhydroxyalkanoat (PHA), insbesondere Poly-3-hydroxybutyrat-co-valerat-Copolymer (PHBV) umfasst.

7. Vorrichtung nach einem der vorhergehenden Ansprüche, wobei der Nistkasten aus einem Rohling (1) gefaltet ist, der aus dem Laminat gebildet ist.

8. Vorrichtung nach einem der Ansprüche 1-7, wobei die Celluloseplatte aus stabiler Pappe mit einem Flächengewicht von 825-925 g/m², vorzugsweise 850-900 g/m² ausgewählt ist, die Schicht aus polymerisiertem Material eine Dicke von 10-80 µm aufweist und das Polymermaterial ausgewählt ist aus Polyethylenterephthalat (PET), vorzugsweise biorientiertem Polyethylenterephthalat (BOPET), oder Polypropylen (PP), vorzugsweise orientiertem Polypropylen (OPP).

9. Verwendung eines Laminats, umfassend eine Celluloseplatte, vorzugsweise stabile Pappe, bedeckt mit einer 10-500 µm dicken Schicht, vorzugsweise einer die Außenseite bedeckenden Schicht, eines Materials, das eine Anzahl von Polymeren umfasst, wobei das Polymermaterial eine Zugfestigkeit von mindestens 25 MPa und eine Eindrück-Mikrohärte von mindestens 80 MPa aufweist, als Konstruktionsmaterial für eine Vorrichtung, wie einen Nistkasten, zum Eingrenzen von Arbeiterinnen der Hummeln, wobei während der Verwendung eine Polymermaterialseite des Laminats vorzugsweise Räumen in der Vorrichtung gegenüberliegt, worin Arbeiterinnen der Hummeln vorhanden sein können.

10. Verwendung nach Anspruch 9, wobei die Dicke der Schicht aus Polymermaterial ausgewählt ist aus 10-20, 10-30, 10-40, 10-50, 10-60, 10-70, 10-80, 10-90, 10-100, 10-110, 10-120, 10-130, 10-140, 10-150, 10-160, 10-170, 10-180, 10-190, 10-200, 10-250, 10-300, 10-350, 10-400, 10-450 µm.

11. Verwendung nach einem der vorhergehenden Ansprüche 9-10, wobei die Celluloseplatte eine Masse von mindestens 200 g/m², vorzugsweise 200-3000 g/m², wie 200-2000 g/m², insbesondere 550-1200 g/m² aufweist.

12. Verwendung nach einem der vorhergehenden Ansprüche 9-11, wobei die Zugfestigkeit einen Wert aufweist, der einen Wert ausgewählt aus 30, 40, 50, 60, 70, 80, 90, 100, 110, 120, 130, 140, 150, 160, 170, 180, 190, 200, 210, 220, 230 MPa übersteigt.

13. Verwendung nach einem der vorhergehenden Ansprüche 9-12, wobei die Eindrück-Mikrohärte einen Wert aufweist, der einen Wert ausgewählt aus 80, 90, 100, 120, 150, 200, 250, 300, 350, 400, 450, 500 MPa übersteigt.

14. Verwendung nach einem der vorhergehenden Ansprüche 9-13, wobei das Polymermaterial ein oder mehrere Polymere ausgewählt aus Polyamid (PA), Polystyrol (PS), Polyethylenterephthalat (PET), Polypropylen (PP), Polyvinylchlorid (PVC), Polymilchsäure (PLA) oder Polyhydroxyalkanoat (PHA), insbesondere Poly-3-hydroxybutyrat-co-valerat-Copolymer (PHBV) umfasst.

15. Verwendung nach einem der vorhergehenden Ansprüche 9-14, wobei ein Rohling (1) aus dem Laminat gebildet und die Vorrichtung aus dem Rohling gefaltet und vorzugsweise der gefaltete Rohling (1) in einer gefalteten Konfiguration fixiert wird.

16. Verwendung nach einem der Ansprüche 9-15, wobei die Celluloseplatte aus stabiler Pappe mit einem Flächengewicht von 825-925 g/m², vorzugsweise 850-900 g/m², ausgewählt ist, die Schicht aus Polymermaterial eine Dicke von 10-80 µm aufweist und das Polymermaterial ausgewählt ist aus Polyethylenterephthalat (PET), vorzugsweise biorientiertem Polyethylenterephthalat (BOPET), oder Polypropylen (PP), vorzugsweise orientiertem Polypropylen (OPP).

17. Verfahren zum Eingrenzen von Arbeiterinnen der Hummeln, umfassend:
- Bereitstellen eines Laminats, umfassend eine Celluloseplatte, vorzugsweise stabile Pappe, bedeckt mit einer 10-500 µm dicken Schicht aus einem Material, das eine Anzahl von Polymeren umfasst, wobei das Polymermaterial eine Zugfestigkeit von mindestens 25 MPa und eine Eindrück-Mikrohärte von mindestens 80 MPa aufweist, wobei eine Schicht des Polymermaterials vorzugsweise eine Außenseitenschicht des Laminats ist;
- Konstruieren einer Vorrichtung, wie eines Nistkastens, umfassend eine Anzahl von eingrenzenden Kammern, die zum Eingrenzen von Arbeiterinnen der Hummeln geeignet sind, aus dem Laminat, wobei die Vorrichtung vorzugsweise derart konstruiert ist, dass eine Polymerschichtseite des Laminats Räumen gegenüber liegt, worin Arbeiterinnen der Hummeln vorhanden sein können;
- Platzieren einer Anzahl von Arbeiterinnen der Hummeln in eingrenzenden Kammern der Vorrichtung.

18. Verfahren nach Anspruch 17, wobei die Vorrichtung eine Vorrichtung nach einem der Ansprüche 1-7 ist.

## Revendications

1. Dispositif, pour confiner des bourdons *(Bombus* spp.) travailleurs, tel qu'un nichoir, comprenant une pluralité de barrières (2, 3, 4, 5) formant une pluralité de chambres adaptées pour confiner des bourdons travailleurs, dans lequel lesdites barrières (2, 3, 4, 5) comprennent un stratifié comprenant un panneau de cellulose, de préférence un panneau en papier solide, recouvert avec une couche de 10 à 500 µm d'épaisseur d'un matériau comprenant une pluralité de polymères, ledit matériau polymère ayant une résistance à la traction d'au moins 25 MPa et une microdureté par pénétration d'au moins 80 MPa, dans lequel une couche de matériau polymère est de préférence une couche de surface externe du stratifié, et dans lequel un côté du stratifié où le matériau polymère est situé est de préférence orienté vers l'espace de la pluralité de chambres où des bourdons travailleurs peuvent être présents.

2. Dispositif selon la revendication 1, dans lequel l'épaisseur de la couche de matériau polymère est choisie parmi 10 à 20, 10 à 30, 10 à 40, 10 à 50, 10 à 60, 10 à 70, 10 à 80, 10 à 90, 10 à 100, 10 à 110, 10 à 120, 10 à 130, 10 à 140, 10 à 150, 10 à 160, 10 à 170, 10 à 180, 10 à 190, 10 à 200, 10 à 250, 10 à 300, 10 à 350, 10 à 400, 10 à 450 µm.

3. Dispositif selon l'une quelconque des revendications précédentes, dans lequel le panneau de cellulose a une masse d'au moins 200 g/m², de préférence 200 à 3000 g/m², telle que 200 à 2000 g/m², plus préférablement de 550 à 1200 g/m².

4. Dispositif selon l'une quelconque des revendications précédentes, dans lequel la résistance à la traction a une valeur supérieure à une valeur choisie parmi 30, 40, 50, 60, 70, 80, 90, 100, 110, 120, 130, 140, 150, 160, 170, 180, 190, 200, 210, 220, 230 MPa.

5. Dispositif selon l'une quelconque des revendications précédentes, dans lequel la microdureté par pénétration a une valeur supérieure à une valeur choisie parmi 80, 90, 100, 120, 150, 200, 250, 300, 350, 400, 450, 500 MPa.

6. Dispositif selon l'une quelconque des revendications précédentes, dans lequel le matériau polymère comprend un ou plusieurs polymères choisis parmi le polyamide (PA), le polystyrène (PS), le polyéthylène téréphtalate (PET), polypropylène (PP), le polychlorure de vinyle (PVC), l'acide polylactique (PLA) ou un polyhydroxyalcanoate (PHA), en particulier un copolymère de poly-3-hydroxybutyratecovalérate (PHBV).

7. Dispositif selon l'une quelconque des revendications précédentes, dans lequel le nichoir est plié à partir d'une préforme (1) formée à partir du stratifié.

8. Dispositif selon l'une quelconque des revendications 1 à 7, dans lequel le panneau de cellulose est choisi en tant que panneau de papier solide ayant un grammage de 825 à 925 g/m², de préférence de 850 à 900 g/m², la couche de matériau polymérisé a une épaisseur de 10 à 80 µm, et le matériau polymère est choisi comme étant un polyéthylène-téréphtalate (PET), de préférence un polyéthylène-téréphtalate bi-orienté (BOPET), ou un polypropylène (PP), de préférence un polypropylène orienté (OPP).

9. Utilisation d'un stratifié comprenant un panneau de cellulose, de préférence un panneau de papier solide, recouverte avec une couche de 10 à 500 µm d'épaisseur, de préférence sous la forme d'une couche de couverture de surface externe, d'un matériau comprenant une pluralité de polymères, ledit matériau polymère ayant une résistance à la traction d'au moins 25 MPa et une microdureté par pénétration d'au moins 80 MPa, en tant que matériau de construction pour un dispositif, tel qu'un nichoir, pour confiner des bourdons travailleurs, où, dans ladite utilisation, un côté matériau polymère du stratifié est de préférence orienté vers des espaces dans le dispositif où des bourdons travailleurs peuvent être présents.

10. Utilisation selon la revendication 9, dans laquelle l'épaisseur de la couche de matériau polymère est choisie parmi 10 à 20, 10 à 30, 10 à 40, 10 à 50, 10 à 60, 10 à 70, 10 à 80, 10 à 90, 10 à 100, 10 à 110, 10 à 120, 10 à 130, 10 à 140, 10 à 150, 10 à 160, 10 à 170, 10 à 180, 10 à 190, 10 à 200, 10 à 250, 10 à 300, 10 à 350, 10 à 400, 10 à 450 µm.

11. Utilisation selon l'une quelconque des revendications précédentes 9 à 10, dans laquelle le panneau de cellulose a une masse d'au moins 200 g/m², de préférence 200 à 3000 g/m², par exemple 200 à 2000 g/m², plus préférablement 550 à 1200 g/m².

12. Utilisation selon l'une quelconque des revendications précédentes 9 à 11, dans laquelle la résistance à la traction a une valeur supérieure à une valeur choisie parmi 30, 40, 50, 60, 70, 80, 90, 100, 110, 120, 130, 140, 150, 160, 170, 180, 190, 200, 210, 220, 230 MPa.

13. Utilisation selon l'une quelconque des revendications précédentes 9 à 12, dans laquelle la microdureté par pénétration a une valeur supérieure à une valeur choisie parmi 80, 90, 100, 120, 150, 200, 250, 300, 350, 400, 450, 500 MPa.

14. Utilisation selon l'une quelconque des revendications précédentes 9 à 13, dans laquelle le matériau polymère comprend un ou plusieurs polymères choisi parmi le polyamide (PA), le polystyrène (PS), le polyéthylène-téréphtalate (PET), le polypropylène (PP), le polychlorure de vinyle (PVC), l'acide polylactique (PLA) ou un polyhydroxyalcanoate (PHA), en particulier un copolymère de poly-3-hydroxybutyratecovalérate (PHBV).

15. Utilisation selon l'une quelconque des revendications précédentes 9 à 14, dans laquelle une préforme (1) est forme à partir du stratifié et le dispositif est plié à partir de la préforme et de préférence la préforme pliée (1) est fixée dans une configuration pliée.

16. Utilisation selon l'une quelconque des revendications 9-15, dans laquelle le panneau de cellulose est choisi en tant que panneau de papier solide ayant un grammage de 825 à 925 g/m², de préférence de 850 à 900 g/m², la couche de matériau polymère a une épaisseur de 10 à 80 µm, et le matériau polymère est choisi comme étant un polyéthylène-téréphtalate (PET), de préférence un polyéthylène-téréphtalate bi-orienté (BOPET) ou un polypropylène (PP), de préférence un polypropylène orienté (OPP).

17. Procédé pour confiner des bourdons travailleurs comprenant :
- la fourniture d'un stratifié comprenant un panneau de cellulose, de préférence un panneau en papier solide, recouvert avec une couche de 10 à 500 µm d'épaisseur d'un matériau comprenant une pluralité de polymères, ledit matériau polymère ayant une résistance à la traction d'au moins 25 MPa et une microdureté par pénétration d'au moins 80 MPa, dans lequel une couche de matériau polymère est de préférence une couche de surface externe du stratifié ;
- la construction à partir du stratifié d'un dispositif, tel qu'un nichoir, comprenant une pluralité de chambres de confinement adaptées pour confiner des bourdons travailleurs, dans lequel le dispositif est de préférence construit de sorte qu'un côté de couche de polymère du stratifié soit orienté vers des espaces dans lesquels des bourdons travailleurs peuvent être présents ;
- le placement d'une pluralité de bourdons travailleurs dans des chambres de confinement du dispositif.

18. Procédé selon la revendication 17, dans lequel le dispositif est un dispositif selon l'une quelconque des revendications 1 à 7.
